# EUROPÄISCHE PATENTSCHRIFT

(11) **EP 3 665 113 B1**
(45) Veröffentlichungstag und Bekanntmachung des Hinweises auf die Patenterteilung: **24.11.2021**
(21) Anmeldenummer: 18779566.1
(22) Anmeldetag: 08.08.2018
(51) Int. Cl.: B65H 35/00, A61F 15/00

(54) **ABROLLVORRICHTUNG**
UNWINDING DEVICE
DISPOSITIF DE DÉROULAGE

(30) Priorität: 08.08.2017 DE 102017118007
(43) Veröffentlichungstag der Anmeldung: 17.06.2020
(73) Patentinhaber: Endriss, Ditte, 89275 Elchingen (DE)
(72) Erfinder: Endriss, Ditte, 89275 Elchingen (DE)
(74) Vertreter: Hentrich Patentanwälte PartG mbB
(86) Internationale Anmeldenummer: PCT/EP2018/071460
(87) Internationale Veröffentlichungsnummer: WO 2019/030259

(56) Entgegenhaltungen:
- CN-U- 203 229 251
- DE-A1- 2 758 563
- DE-U1-202013 007 461
- JP-U- S 633 052
- US-A- 5 392 676
- US-A1- 2015 090 849

## Beschreibung

Die Erfindung betrifft eine Abrollvorrichtung für eine Kleberolle oder für ein auf einer Rolle gewickeltes Medizinprodukt, mit einer Grundplatte, an der ein Zapfen zur Aufnahme der Rolle oder der Kleberolle und ein von dem Zapfen beabstandetes Abrisselement mit einer Abrissklinge angebracht sind, und mit einem mit der Grundplatte verbundenen Befestigungsabschnitt zum zumindest teilweisen Umgreifen einer Stange, einer Leiste oder dergleichen.

Eine Abrollvorrichtung ist beispielsweise aus der DE 20 2014 103 429 U1 bekannt. Nachteilig bei Verwendung dieser Abrollvorrichtung für gewickelte Medizinprodukte, wie Pflaster, Mullbinden, Verbandsmaterial etc. ist, dass das Infektionsrisiko aufgrund der Handhabung erhöht ist. Um ein Medizinprodukt von der Abrollvorrichtung abzutrennen, muss eine Hand immer die Abrollvorrichtung und die andere Hand immer das Klebeband bzw. das Medizinprodukt berühren. Auf diese Weise gelangen Bakterien, Viren und auch multiresistente Erreger von der Hand auf die Abrollvorrichtung. Somit können die Erreger bei jeder Verwendung der Abrollvorrichtung auf Patienten ungewollt übertragen werden.

Weitere - nachteilige - aus dem Stand der Technik bekannte Abrollvorrichtungen sind in der DE 10 2004 018 618 A1 oder der DE 197 31 428 A1 beschrieben.

Die EP 0 963 933 A1 und die US 5 392 676 A offenbaren jeweils eine Abrollvorrichtung gemäß dem Oberbegriff des Anspruchs 1.

Die Aufgabe der Erfindung ist es daher, eine Abrollvorrichtung der eingangs genannten Art dahingehend weiterzubilden, dass eine hygienischere und sichere Handhabung ermöglicht wird.

Die Aufgabe der Erfindung wird mit einer Abrollvorrichtung mit dem Merkmalsbestand des Anspruchs 1 gelöst. Vorteilhafte Ausgestaltungen mit zweckmäßigen Weiterbildungen der Erfindung sind in den abhängigen Ansprüchen angegeben.

Insbesondere umfasst der Befestigungsabschnitt einen mit der Grundplatte verbundenen Schenkel, der in einen Steg übergeht, welcher mit einem freien Schenkel verbunden ist, derart, dass zwischen dem freien Schenkel und dem verbundenen Schenkel die Stange, die Leiste oder dergleichen aufgenommen werden kann, wobei mindestens ein Fixierungselement vorhanden ist, um den verbundenen Schenkel mit dem freien Schenkel zu verbinden. Durch die entsprechende Ausbildung des Befestigungsabschnitts zum zumindest teilweisen Umgreifen einer Stange, einer Leiste oder dergleichen ist eine einhändige Bedienbarkeit ermöglicht, wobei er die Abrollvorrichtung betriebssicher an der Stange, der Leiste oder dergleichen fixiert. Die Abrollvorrichtung ist vorzugsweise an einer Infusionsstange, an einem Bett, einer Wandleiste oder dergleichen - beispielsweise durch Einhängen - befestigbar. Lediglich der Streifen, der von der Rolle abgerissen wird, kommt mit der Hand in Berührung. Die Übertragung von Bakterien oder Keimen auf die Abrollvorrichtung oder auf die Rolle bzw. Kleberolle wird so erheblich reduziert. Ein auf einer Rolle gewickeltes Medizinprodukt umfasst dabei Pflaster, Mullbinden oder jegliches anderes Verbandsmaterial. In einer bevorzugten Ausführungsform ist die Abrissklinge mit Abrisszähnen gebildet.

Um eine winkelfeste Lage der Abrollvorrichtung erzielen zu können, hat es sich als vorteilhaft herausgestellt, wenn zwei der Fixierungselemente vorgesehen sind, die den freien Schenkel mit dem verbundenen Schenkel jeweils randseitig miteinander verbinden.

Eine sichere Fixierung wird zudem dadurch erreicht, dass das mindestens eine Fixierungselement den freien Schenkel mit dem verbundenen Schenkel derart verbindet, dass mittels des freien Schenkels, des Stegs und des verbundenen Schenkels ein Aufnahmeraum zur Aufnahme oder für einen Durchtritt der Stange ausgebildet ist, welcher von dem mindestens einen Fixierungselement begrenzt wird.

Zur raschen und einfachen Befestigung der Abrollvorrichtung ist es, insbesondere vorgesehen, dass der Befestigungsabschnitt als ein Klemmelement zur Klemmung der Stange, der Leiste oder dergleichen gebildet ist, insbesondere, dass das mindestens eine Fixierungselement den freien Schenkel mit dem verbundenen Schenkel derart verbindet, dass eine in dem Befestigungsabschnitt aufgenommene Stange, Leiste oder dergleichen, geklemmt ist.

Zur einfacheren Handhabung der Abrollvorrichtung ist es insbesondere vorgesehen, dass der Befestigungsabschnitt bevorzugt als ein Bogen oder als eine Nut gebildet ist. Dabei kann der freie Schenkel parallel oder konkav zur Grundplatte ausgebildet sein.

Um die Abrollvorrichtung an Stangen, Leisten oder dergleichen mit unterschiedlichem Durchmesser bzw. Breite anbringen zu können, ist es vorteilhaft, wenn der freie Schenkel und der mit der Grundplatte verbundene Schenkel ausgestaltet sind, einen Adapter zwischen sich aufzunehmen, um einen Innenumfang des Befestigungsabschnitts anzupassen. Dabei ist es insbesondere bevorzugt, wenn der Außendurchmesser des Adapters ungefähr dem Innendurchmesser des Befestigungsabschnitts entspricht. Alternativ oder ergänzend kann der Adapter auch aus einem gegenüber dem Befestigungsabschnitt verschiedenen Material gebildet sein. Beispielsweise kann der Adapter aus einem elastischen Material gebildet sein, um die Stange vor Kratzern und dergleichen zu schützen oder eine Anti-Rutsch-Beschichtung aufweisen, sodass die Abrollvorrichtung an der Stange, einer Leiste oder dergleichen haftet. Die Form des Adapters ist dabei bevorzugt an die Form des Befestigungsabschnitts angepasst: Ist der Befestigungsabschnitt als ein Bogen gebildet, so ist der Adapter auch als ein Bogen gebildet. Der Adapter ist dabei vorzugsweise derart ausgebildet, dass eine Länge des Befestigungsabschnitts mit einer Länge des Adapters übereinstimmt, sich der Adapter also über die Länge des Befestigungsabschnitts vollständig erstreckt. Alternativ ist es möglich, dass der Adapter kürzer ausgestaltet ist als die Länge des Befestigungsabschnitts, oder dass der Adapter unterschiedliche Innendurchmesser aufweist.

Zur lösbaren Verbindung des Adapters mit dem Befestigungsabschnitt ist es bevorzugt, wenn der Befestigungsabschnitt eine Aufnahme aufweist, in die ein am Adapter ausgebildetes Befestigungsglied aufnehmbar ist. Das Befestigungsglied kann dabei beispielsweise als ein Rastglied gebildet sein, welches in der Aufnahme einrastet. Dabei ist es insbesondere vorgesehen, dass die Aufnahme am freien Schenkel und/oder am mit der Grundplatte verbundenen Schenkel ausgebildet ist. Alternativ ist es möglich, dass die Aufnahme am Steg ausgebildet ist. In einer bevorzugten Ausführungsform ist jeweils eine Aufnahme am freien Schenkel und am Schenkel ausgebildet. Besonders bevorzugt ist es, wenn die Aufnahme als ein Einschnitt und das Befestigungsglied als eine zum Einschnitt korrespondierende Schiene gebildet ist, die in den Einschnitt hinein schiebbar ist.

Zur Verbesserung der Hygiene ist es bevorzugt, wenn die Grundplatte zwischen dem Zapfen und dem Abrisselement eine Ausnehmung aufweist. Hierdurch werden die Berührungspunkte der Hand und/oder des Medizinprodukts/der Kleberolle mit der Abrollvorrichtung reduziert und damit die Infektionsgefahr verringert.

In diesem Zusammenhang ist es insbesondere vorgesehen, dass die Grundplatte an dem dem Schenkel gegenüberliegenden Ende eine Kante aufweist, und dass die Ausnehmung sich bis zur Kante erstreckt. Hierdurch wird neben der verbesserten Hygiene auch Material eingespart und das Gewicht reduziert.

Um das Heruntergleiten der Rolle bzw. der Kleberolle zu verhindern ist es bevorzugt, wenn der Zapfen einen Bund aufweist. Dieser kann aus demselben Material wie der Zapfen gebildet sein, beispielsweise aus Kunststoff, oder aus einem elastischen Material. Wird in einer alternativen Ausführungsform ein elastisches Material für den Bund gewählt, so wird er beim darüber Schieben der (Klebe-)Rolle komprimiert. Ist die (Klebe-)Rolle vollständig über den Bund geschoben worden, so dekomprimiert dieser wieder und fixiert so die (Klebe-)Rolle an dem Zapfen. Der Durchmesser des Bundes ist dabei größer gewählt, als der Innendurchmesser der (Klebe-)Rolle. Alternativ oder ergänzend kann die Kleberolle oder die Rolle durch den elastischen Bund mit der Grundplatte geklemmt werden. In einer alternativen Ausführungsform ist der Bund nur teilweise aus elastischem Material gefertigt, wobei bevorzugt ein äußerer Ring des Bundes aus einem elastischen Material gefertigt ist. In einer weiteren alternativen Ausführungsform können der Zapfen und der Bund auch zweiteilig gebildet sein, sodass der Bund auf den Zapfen gesteckt werden kann. Die Rolle bzw. die Kleberolle kann auf dem Zapfen aufgenommen werden, indem der Bund vom Zapfen gelöst wird, die Rolle aufgesteckt wird, und dann der Bund wieder auf den Zapfen gesteckt wird.

Zur sicheren Fixierung der Abrollvorrichtung an der Stange, der Leiste oder dergleichen, ist es insbesondere vorgesehen, dass das mindestens eine Fixierungselement als eine Schraube gebildet ist, die durch eine in einem ersten der Schenkel ausgebildete Öffnung geführt ist, und die mit einem einem zweiten der Schenkel zugeordneten Gewinde verschraubt ist oder verschraubt wird. Das Fixierungselement kann alternativ gebildet sein als ein Bolzen, als eine Mutter oder als ein Sperrbolzen.

Zur sicheren Fixierung der Abrolleinrichtung entlang der Stange, der Leiste oder dergleichen, ist zudem die Möglichkeit eröffnet, dass in dem zweiten der Schenkel ebenfalls eine Öffnung ausgebildet ist, durch die ein (Gewinde-)Abschnitt der Schraube geführt ist, welcher mit einer Mutter verschraubt ist.

In diesem Zusammenhang ist es sinnvoll, wenn in dem zweiten der Schenkel auf seiner dem ersten der Schenkel abgewandten Seite eine Hinterschneidung ausgebildet ist, die ausgestaltet ist, die Mutter im Wesentlichen drehfest gegenüber dem zweiten der Schenkel zu lagern. Die Mutter dient dann als Kontermutter für die Schraube, wobei die Hinterschneidung auch derart ausgestaltet sein kann, dass die Mutter nicht über die Fläche des verbundenen Schenkels hervorsteht. Eine einfach festzuziehende oder zu lösende manuelle Fixierung der Abrollvorrichtung an der Stange, der Leiste oder dergleichen, lässt sich dadurch realisieren, dass das mindestens eine Fixierungselement als eine Flügelschraube gebildet ist.

Alternativ oder ergänzend ist es auch möglich, dass die Grundplatte eine Bohrung aufweist, durch die die Grundplatte an einer Wand, einer Tür oder dergleichen fixierbar ist.

Eine besonders stabile Abrollvorrichtung ist dadurch gekennzeichnet, dass die Grundplatte und der den freien Schenkel, den Steg und den verbundenen Schenkel umfassende Befestigungsabschnitt einstückig gebildet sind. Hierbei können Fertigungsverfahren wie Spritzgießen oder 3D-Druck in Betracht kommen.

Im Folgenden wird die Erfindung an einem in den Zeichnungen dargestellten Ausführungsbeispiel näher erläutert; es zeigen:
- Fig. 1: eine perspektivische Darstellung der erfindungsgemäßen Abrollvorrichtung;
- Fig. 2: eine Vorderansicht der erfindungsgemäßen Abrollvorrichtung und
- Fig. 3: den Schnitt III-III aus Fig. 2.

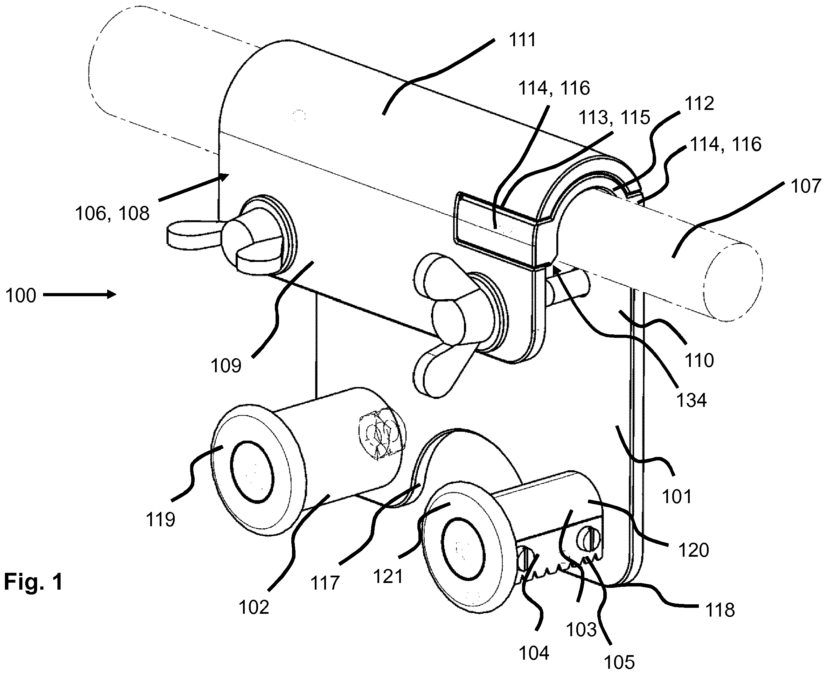

Fig. 1 zeigt eine perspektivische Ansicht der erfindungsgemäßen Abrollvorrichtung 100 für eine nicht gezeigte Kleberolle oder für ein nicht gezeigtes auf einer Rolle gewickeltes Medizinprodukt wie ein Pflaster, eine Mullbinde, Verbandsmaterial, etc..

Die Abrollvorrichtung 100 weist eine Grundplatte 101 auf, an der ein Zapfen 102 zur Aufnahme der Rolle oder der Kleberolle und ein vom Zapfen 102 beabstandetes Abrisselement 103 mit einer Abrissklinge 104 angebracht sind. Auf den Zapfen 102 kann eine Kleberolle, z.B. eine Pflasterrolle aufgesteckt werden. Um die Rolle einfacher abwickeln zu können, ist der Zapfen 104 rotationssymmetrisch entlang seiner Längsachse gebildet. Die Abrissklinge 104 weist für ein einfacheres Abreißen des Klebebands oder des Medizinprodukts Abrisszähne 105 auf. Die Grundplatte 101 umfasst darüber hinaus einen Befestigungsabschnitt 106 zum teilweisen Umgreifen einer Stange 107. Der Befestigungsabschnitt 106 ist dabei als ein Klemmelement 108 zur Klemmung der Stange 107 gebildet. Er umfasst einen freien Schenkel 109 und einen mit der Grundplatte 101 verbundenen Schenkel 110, sowie einen den freien Schenkel 109 mit dem Schenkel 110 verbindenden Steg 111. Im vorliegenden Ausführungsbeispiel ist der Steg 111 als ein Bogen gebildet.

Die Abrollvorrichtung 100 zeichnet sich dadurch aus, dass mindestens ein Fixierungselement 124 vorhanden ist, um den verbundenen Schenkel 109 zu verbinden, vorliegend insbesondere aber dadurch, dass zwei der Fixierungsmittel 124 vorgesehen sind, die den freien Schenkel 109 mit dem verbundenen Schenkel 110 jeweils randseitig miteinander verbinden, zudem dadurch, dass das mindestens eine Fixierungselement 124 den freien Schenkel 109 mit dem verbundenen Schenkel 110 derart verbindet, dass aus dem freien Schenkel 109, dem Steg 111 und dem verbundenen Schenkel 110 ein Aufnahmeraum 134 zur Aufnahme oder für einen Durchtritt der Stange 107 ausgebildet ist, welcher von dem mindestens einen Fixierungselement 124 begrenzt wird.

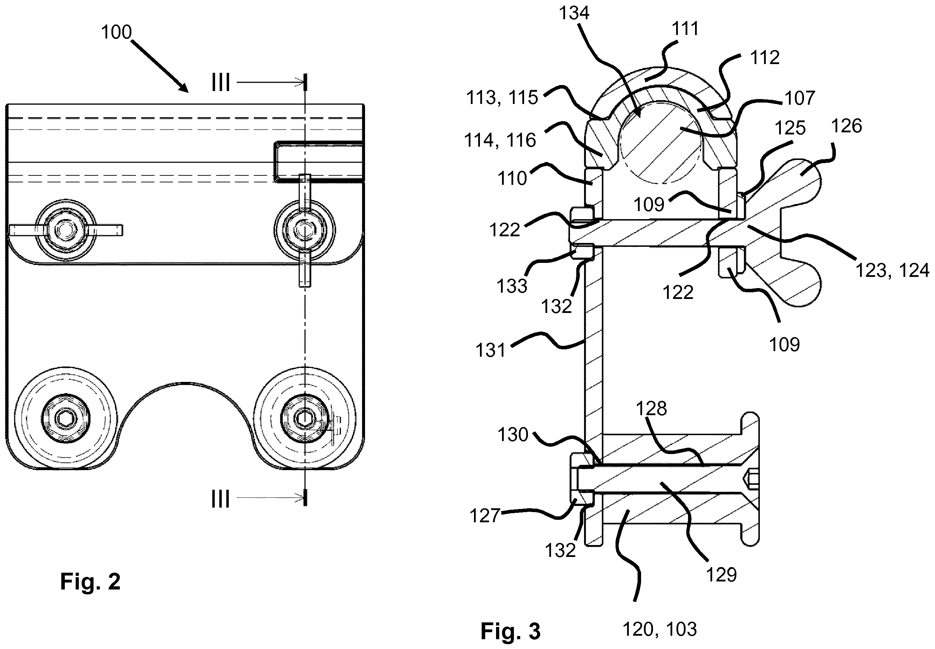

Im gezeigten Ausführungsbeispiel ist zwischen dem Schenkel 110 und dem freien Schenkel 109 ein Adapter 112 aufgenommen, dessen Außendurchmesser angepasst ist an den Innendurchmesser des Befestigungsabschnitts 106. Der Innendurchmesser des Adapters 112 kann dann an beliebige Durchmesser der aufzunehmenden Stange 107 angepasst sein, wobei vorzugsweise die Klemmwirkung erhöht wird. Dabei weist der Befestigungsabschnitt 106 an seinem freien Schenkel 109 und an seinem mit der Grundplatte 101 verbundenen Schenkel 110 je eine Aufnahme 113 auf, in die jeweils ein am Adapter 112 lateral ausgebildetes Befestigungsglied 114 aufnehmbar ist. Dadurch ist eine lösbare Verbindung des Adapters 112 mit dem Befestigungsabschnitt 106 realisiert. Die Aufnahme 113 ist dabei als ein Einschnitt 115 gebildet und das Befestigungsglied 114 als eine zum Einschnitt 115 korrespondierende Schiene 116. Die Schiene 116 ist derart angepasst, um in den Einschnitt 115 eingeschoben zu werden. Die Wechselwirkung zwischen dem Einschnitt 115 und der Schiene 116 ist in Fig. 3 zusätzlich verdeutlicht. Vorzugsweise ist die Länge des Adapters 112 angepasst an die Breite der Abrollvorrichtung 100. Die Länge des Adapters kann aber weniger als 100 Prozent der sich entlang der Stange 107 erstreckenden Breite der Abrollvorrichtung 100 betragen, weiterhin vorzugsweise höchstens zwischen 70 und 90 Prozent der Breite der Vorrichtung 100, weiterhin vorzugsweise zwischen 50 und 70 Prozent der Breite der Vorrichtung, oder vorzugsweise zwischen 20 und 50 Prozent der Breite der Vorrichtung 100. Somit kann die Vorrichtung 100 an Stangen 107 befestigt werden, die entlang ihrer Länge unterschiedliche Durchmesser besitzen.

An der Grundplatte 101 ist zwischen dem Zapfen 102 und dem Abrisselement 103 eine Ausnehmung 117 ausgeprägt. Die Grundplatte 101 weist dabei an dem dem Schenkel 110 gegenüberliegenden Ende eine Kante 118 auf, an welcher die halbkreisförmige Ausnehmung 117 ausgebildet ist. Hierdurch wird die Berührungsfläche der Hände und der (Klebe-)Rolle mit der Abrollvorrichtung 100 minimiert, womit das Infektionsrisiko reduziert ist.

Außerdem weist der Zapfen 102 einen Bund 119 auf, durch den die aufzunehmende Rolle an der Abrollvorrichtung 100 gesichert wird. In einer alternativen Ausführungsform ist der Bund 119 aus einem elastischen Material gebildet, so dass bei der Aufnahme der (Klebe-) Rolle, diese über den elastischen Bund 119 gepresst werden kann bis sie ihre gewünschte Position am Zapfen 102 erreicht hat. Beim Aufschieben der Rolle wird der Bund 119 komprimiert und wenn die Rolle auf dem Zapfen 102 geschoben ist, wieder dekomprimiert. Dies verhindert ein Heruntergleiten der (Klebe-)Rolle vom Zapfen 102 und ermöglicht eine vereinfachte Handhabung.

Das Abrisselement 103 ist als ein zweiter Zapfen 120 gebildet, der einen zweiten Bund 121 aufweist, wobei dieser aus demselben Material gebildet ist, wie der erste Zapfen 102, bevorzugt nämlich aus Kunststoff. Die Abrissklinge 104 ist mit Befestigungsmitteln, im gezeigten Beispiel mittels zwei Schrauben, am Abrisselement 103 bzw. am weiteren Zapfen 120 festgelegt. Diese lösbare Befestigung der Abrissklinge 104 am Abrisselement 103 ermöglicht ein einfaches Austauschen der Abrissklinge 104, sollte diese infolge intensiver Nutzung abgenutzt oder stumpf geworden sein.

Die Fixierung der Abrollvorrichtung 100 an der Stange 107 erfolgt zum einen, indem der Befestigungsabschnitt 106 diese umgreift und zum anderen indem am freien Schenkel 109 und am mit der Grundplatte 101 verbundenen Schenkel 110 jeweils eine Öffnung 122 in Form eines Bohrlochs ausgebildet ist. In der Öffnung 122 ist ein als eine Flügelschraube 123 gebildetes Fixierungselement 124 angeordnet.

Fig. 3 zeigt den Schnitt III-III aus Fig. 2 der erfindungsgemäßen Abrollvorrichtung 100. Dabei wird insbesondere der Befestigungsmechanismus der Abrollvorrichtung 100 an die Stange 107 verdeutlicht. Zwischen dem Fixierungselement 124 und dem freien Schenkel 109 ist eine Unterlegscheibe 125 angeordnet. Weiterhin weist die Flügelschraube 123 an dem den Flügeln 126 abgewandten Ende eine Mutter 133 auf, so dass zwischen den Flügeln 126 der Flügelschraube 123 und der Mutter 133, der freie Schenkel 109 und der mit der Grundplatte 101 verbundene Schenkel 110 angeordnet sind. Durch Verdrehen der Flügelschraube 123 kann die Stange 107 im Befestigungsabschnitt 106 fixiert bzw. geklemmt oder auch gelöst werden. Die Flügelschraube 123 kann also auf der Rückseite 131 der Grundplatte 101 mit einer (Konter-)Mutter 133 verschraubt werden. Um zu verhindern, dass sich die Mutter 133 beim Verdrehen der Flügelschraube 123 mitdreht, sollte diese drehfest gelagert werden. Das gezeigte Ausführungsbeispiel sieht deshalb an der Rückseite 131 Hinterschneidungen 132 vor, die in ihren Abmessungen näherungsweise angepasst sind an die Abmessungen der Mutter 133. Diese Hinterschneidungen 132 sind ausgestaltet, ein Verdrehen oder ein Mitdrehen der Mutter 133 zu verhindern. Um die Stange 107 zu klemmen genügt es also ausschließlich die Flügelschraube 123 zu verdrehen aufgrund der selbsthemmenden Lage der Mutter 133. Dies ist auch in denjenigen Fällen von Vorteil, in denen die Rückseite 131 der Grundplatte 101 nicht zugänglich ist, um die Mutter 133 (manuel) zu kontern.

Darüber hinaus zeigt Fig. 3 die Befestigung des zylinderförmigen zweiten Zapfens 120 bzw. des Abrisselements 103 an der Grundplatte 101. Das Abrisselement 103 und auch der in Fig. 3 nicht gezeigte Zapfen 102 weisen einen durchgehenden Tunnel 128 auf, in der eine Schraube 129 oder dergleichen aufgenommen ist. An der Grundplatte 101 sind Bohrungen 130 ausgebildet, so dass das Abrisselement 103 und der nicht gezeigte Zapfen 102 mittels der Schraube 130 und einer Mutter 127 mit der Grundplatte 101 fixierbar sind. Hierdurch wird es auch ermöglicht den Zapfen 102 und/oder das Abrisselement 103 bei Beschädigung oder starker Abnutzung zu ersetzen. Auch hier sind Hinterschneidungen 132 an der Rückseite 131 der Grundplatte 101 ausgeprägt, in die die Muttern 127 einsetzbar sind um die Drehung der Schraube 129 zu kontern.

Durch die Fixierung der Abrollvorrichtung 100 an der Stange 107 mittels des Befestigungsabschnitts 106 und durch die Fixierungselemente 124 ist eine einhändige Bedienbarkeit der Abrollvorrichtung 100 möglich. Eine Verunreinigung der Abrollvorrichtung 100, der Rolle oder der Kleberolle wird so verhindert, da die Hände nur mit dem Teil der Kleberolle oder des auf einer Rolle gewickelten Medizinprodukts in Berührung kommt, welches abgerissen wird.

### Bezugszeichenliste

- 100: Abrollvorrichtung
- 101: Grundplatte
- 102: Zapfen
- 103: Abrisselement
- 104: Abrissklinge
- 105: Abrisszähne
- 106: Befestigungsabschnitt
- 107: Stange
- 108: Klemmelement
- 109: freier Schenkel
- 110: Schenkel
- 111: Steg
- 112: Adapter
- 113: Aufnahme
- 114: Befestigungsglied
- 115: Einschnitt
- 116: Schiene
- 117: Ausnehmung
- 118: Kante
- 119: Bund
- 120: zweiter Zapfen
- 121: zweiter Bund
- 122: Öffnung
- 123: Flügelschraube
- 124: Fixierungselement
- 125: Unterlegscheibe
- 126: Flügel
- 127: Mutter
- 128: Tunnel
- 129: Schraube
- 130: Bohrung
- 131: Rückseite
- 132: Hinterschneidung
- 133: Mutter
- 134: Aufnahmeraum

## Patentansprüche

1. Abrollvorrichtung (100) für eine Kleberolle oder für ein auf einer Rolle gewickeltes Medizinprodukt, mit einer Grundplatte (101), an der ein Zapfen (102) zur Aufnahme der Rolle oder der Kleberolle und ein von dem Zapfen (102) beabstandetes Abrisselement (103) mit einer Abrissklinge (104) angebracht sind, und mit einem mit der Grundplatte (102) verbundenen Befestigungsabschnitt (106) zum zumindest teilweisen Umgreifen einer Stange (107), einer Leiste oder dergleichen, **dadurch gekennzeichnet, dass** der Befestigungsabschnitt (106) einen mit der Grundplatte (101) verbundenen Schenkel (110) umfasst, der in einen Steg (111) übergeht, welcher mit einem freien Schenkel (109) verbunden ist, derart, dass zwischen dem freien Schenkel (109) und dem verbundenen Schenkel (110) die Stange (107), die Leiste oder dergleichen aufgenommen werden kann, und dass mindestens ein Fixierungselement (124) vorhanden ist, um den verbundenen Schenkel (110) mit dem freien Schenkel (109) zu verbinden.

2. Abrollvorrichtung (100) nach Anspruch 1, **dadurch gekennzeichnet, dass** zwei der Fixierungselemente (124) vorgesehen sind, die den freien Schenkel (109) mit dem verbundenen Schenkel (110) jeweils randseitig miteinander verbinden.

3. Abrollvorrichtung (100) nach Anspruch 1 oder 2, **dadurch gekennzeichnet, dass** das mindestens eine Fixierungselement (124) den freien Schenkel (109) mit dem verbundenen Schenkel (110) derart verbindet, dass mittels des freien Schenkels (109), des Stegs (111) und des verbundenen Schenkels (110) ein Aufnahmeraum (134) zur Aufnahme oder für einen Durchtritt der Stange (107) ausgebildet ist, welcher von dem mindestens einen Fixierungselement (124) begrenzt wird.

4. Abrollvorrichtung (100) nach einem der Ansprüche 1 bis 3, **dadurch gekennzeichnet, dass** das mindestens eine Fixierungselement (124) den freien Schenkel (109) mit dem verbundenen Schenkel (110) derart verbindet, dass eine in dem Befestigungsabschnitt (106) aufgenommene Stange (107), Leiste oder dergleichen, geklemmt ist.

5. Abrollvorrichtung (100) nach einem der Ansprüche 1 bis 4, **dadurch gekennzeichnet, dass** das mindestens eine Fixierungselement (124) als eine Schraube gebildet ist, die durch eine in einem ersten der Schenkel (109, 110) ausgebildete Öffnung (122) geführt ist, und die mit einem einem zweiten der Schenkel (109, 110) zugeordneten Gewinde verschraubt ist oder verschraubt wird.

6. Abrollvorrichtung (100) nach Anspruch 5, **dadurch gekennzeichnet, dass** in dem zweiten der Schenkel (109, 110) ebenfalls eine Öffnung (122) ausgebildet ist, durch die ein Abschnitt der Schraube geführt ist, welcher mit einer Mutter (127) verschraubt ist.

7. Abrollvorrichtung (100) nach Anspruch 6, **dadurch gekennzeichnet, dass** in dem zweiten der Schenkel (109, 110) auf seiner dem ersten der Schenkel (109, 110) abgewandten Seite eine Hinterschneidung (132) ausgebildet ist, die ausgestaltet ist, die Mutter (127) im Wesentlichen drehfest gegenüber dem zweiten der Schenkel (109, 110) zu lagern.

8. Abrollvorrichtung (100) nach einem der Ansprüche 1 bis 7, **dadurch gekennzeichnet, dass** das mindestens eine Fixierungselement (124) als eine Flügelschraube (123) gebildet ist.

9. Abrollvorrichtung (100) nach einem der Ansprüche 1 bis 8, **dadurch gekennzeichnet, dass** der freie Schenkel (109) und der verbundene Schenkel (110) ausgestaltet sind, einen Adapter (112) zwischen sich aufzunehmen, um einen Innenumfang des Befestigungsabschnitts (106) anzupassen.

10. Abrollvorrichtung (100) nach Anspruch 9, **dadurch gekennzeichnet, dass** der Adapter (112) sich über die Länge des Befestigungsabschnitts (106) vollständig erstreckt.

11. Abrollvorrichtung (100) nach Anspruch 9 oder 10, **dadurch gekennzeichnet, dass** der Befestigungsabschnitt (106) eine Aufnahme (113) aufweist, in die ein am Adapter (112) ausgebildetes Befestigungsglied (114) aufnehmbar ist zur lösbaren Verbindung des Adapters (112) mit dem Befestigungsabschnitt (106).

12. Abrollvorrichtung (100) nach einem der Ansprüche 1 bis 11, **dadurch gekennzeichnet, dass** die Grundplatte (101) zwischen dem Zapfen (102) und dem Abrisselement (103) eine Ausnehmung (117) aufweist.

13. Abrollvorrichtung (100) nach Anspruch 12, **dadurch gekennzeichnet, dass** die Grundplatte (101) an dem dem Schenkel (110) gegenüberliegenden Ende eine Kante (118) aufweist und dass die Ausnehmung (117) sich bis zur Kante (118) erstreckt.

14. Abrollvorrichtung (100) nach einem der Ansprüche 1 bis 13, **dadurch gekennzeichnet, dass** der Zapfen (102) einen Bund (119) aufweist, welcher aus einem elastischen Material gebildet ist.

15. Abrollvorrichtung (100) nach einem der Ansprüche 1 bis 14, **dadurch gekennzeichnet, dass** die Grundplatte (101) und der den freien Schenkel (109), den Steg (111) und den verbundenen Schenkel (110) umfassende Befestigungsabschnitt (106) einstückig gebildet sind.

## Claims

1. Dispensing device (100) for an adhesive roll or for a medical product wound on a roll, having a base plate (101), to which a pin (102) for receiving the roll or the adhesive roll and a tearing section (103), which is spaced apart from the pin (102) and has a tearing blade (104), are attached, and having a fastening section (106), which is connected to the base plate (101) and is intended for at least partially gripping around a rod (107), a strip or the like, characterized that the fastening section (106) comprises an arm (110) connected to the base plate (101), which arm (110) merges into a crosspiece (111) connected to a free arm (109), such that between the free arm (109) and the connected arm (110) the rod (107), the ledge or the like can be received, and that at least one fastening unit (124) is provided to connect the connected arm (110) to the free arm (109).

2. The dispensing device (100) according to claim 1, **characterized in that** two fastening units (124) are provided, joining the free arm (109) with the connected arm (110) at edges, respectively.

3. The dispensing device (100) according to claim 1 or 2, **characterized in that** the at least one fastening unit (124) joins the free arm (109) to the connected arm (110) in such a way as to form a receiving space (134) between the free arm (109), the crosspiece (111) and the connected arm (110) for receiving or for providing a passage to the rod (107) delimited by at least the one fastening unit (124).

4. The dispensing device (100) according to one of the claims 1 to 3, **characterized in that** the at least one fastening unit (124) joins the free arm (109) to the connected arm (110) in such a way as to clamp the rod (107) or bar received in the fastening section (106).

5. The dispensing device (100) according to any one of the claims 1 to 4, **characterized in that** the at least one fastening unit (124) is provided in the form of a screw, which is passed through an aperture formed in a first of the arms (109, 110) and which is screwed or bolted to a thread corresponding to a second of the arms (109, 110).

6. The dispensing device (100) according to claim 5, **characterized in that** in the second of the arms (109, 110) also comprises an aperture (122), through which a section of the screw is passed, which is screwed with a nut (127).

7. The dispensing device (100) according to claim 6, **characterized in that** the second of the arms (109, 110) comprises an undercut (132) on the side facing away from the first of the arms (109,110), designed to mount the nut (127) essentially in a non-rotational manner relative to the second of the arms (109, 110).

8. The dispensing device (100) according to one of the claims 1 to 7, **characterized in that** at least one fastening unit (124) is formed as a wing screw (123).

9. The dispensing device according to any one of the claims 1 to 8, **characterized in that** the free arm (109) and the connected arm (110) allow for accommodation of an adapter (112) between them, to adjust an inner diameter of the fastening section (106).

10. The dispensing device (100) according to claim 9, **characterized in that** the adapter (112) extends throughout the entire length of the fastening section (106).

11. The dispensing device (100) according to claim 9 or 10, **characterized in that** the fastening section (106) comprises a seat (113) allowing for accommodation of a mounting unit (114) attached to the adapter (112) and thus facilitating for a detachable connection between the adapter (112) and fastening section (106).

12. The dispensing device (100) according to any one of the claims 1 to 11, **characterized in that** the base plate (101) comprises an indentation (117) between the pin (102) and the tearing section (103).

13. The dispensing device (100) according to claim 12, **characterized in that** the base plate (101) comprises an edge (118) at the opposite end of the arm (110) and wherein the indentation (117) extends to the edge (118).

14. The dispensing device (100) according to any one of the claims 1 to 13, **characterized in that** the pin (102) comprises a flange (119) made of an elastic material.

15. The dispensing device (100) according to any one of the claims 1 to 14, **characterized in that** the base plate (101) and the fastening section (106) comprising the free arm (109), the crosspiece (111) and the connected arm (110) are formed as one piece.

## Revendications

1. Dispositif de déroulement (100) pour un rouleau adhésif ou pour un produit médical enroulé sur un rouleau, avec une plaque de base (101) sur laquelle sont disposés un tenon (102) pour recevoir le rouleau ou le rouleau adhésif et un élément de déchirure (103) espacé du tenon (102) avec une lame de déchirure (104), et avec une section de fixation (106) reliée à la plaque de base (102) pour enserrer au moins partiellement une tige (107), une moulure ou similaire, **caractérisé en ce que** la section de fixation (106) comprend une branche (110) reliée à la plaque de base (101) et se raccordant à une traverse (111) reliée à une branche libre (109), de telle sorte que la tige (107), la moulure ou similaire puisse être reçue entre la branche libre (109) et la branche reliée (110), et **en ce qu'**au moins un élément de fixation (124) est prévu pour relier la branche reliée (110) à la branche libre (109).

2. Dispositif de déroulement (100) selon la revendication 1, **caractérisé en ce que** deux des éléments de fixation (124) sont prévus, qui relient chacun respectivement côté bord la branche libre (109) et la branche reliée (110).

3. Dispositif de déroulement (100) selon la revendication 1 ou 2, **caractérisé en ce que** le au moins un élément de fixation (124) relie la branche libre (109) à la branche reliée (110) de telle sorte qu'un espace de réception (134) pour recevoir ou pour un passage de la tige (107) soit formé au moyen de la branche libre (109), de la traverse (111) et de la branche reliée (110), lequel espace de réception est limité par le au moins un élément de fixation (124).

4. Dispositif de déroulement (100) selon l'une quelconque des revendications 1 à 3, **caractérisé en ce que** le au moins un élément de fixation (124) relie la branche libre (109) à la branche reliée (110) de telle sorte qu'une tige (107), une moulure ou similaire logée dans la section de fixation (106) soit serrée.

5. Dispositif de déroulement (100) selon l'une quelconque des revendications 1 à 4, **caractérisé en ce que** le au moins un élément de fixation (124) est réalisé sous la forme d'une vis qui est guidée à travers une ouverture (122) formée dans une première des branches (109, 110) et qui est vissée ou sera vissée dans un taraudage associé à une seconde des branches (109, 110).

6. Dispositif de déroulement (100) selon la revendication 5, **caractérisé en ce que**, dans la seconde des branches (109, 110), une ouverture (122) est également formée à travers laquelle est passée une partie de la vis, laquelle partie est vissée dans un écrou (127).

7. Dispositif de déroulement (100) selon la revendication 6, **caractérisé en ce qu'**une contre-dépouille (132) est formée dans la seconde des branches (109, 110) sur son côté opposé à la première des branches (109, 110), laquelle contre-dépouille est destinée à monter l'écrou (127) de manière sensiblement fixe en rotation par rapport à la seconde des branches (109, 110).

8. Dispositif de déroulement (100) selon l'une quelconque des revendications 1 à 7, **caractérisé en ce que** le au moins un élément de fixation (124) est réalisé sous la forme d'une vis à oreilles (123).

9. Dispositif de déroulement (100) selon l'une quelconque des revendications 1 à 8, **caractérisé en ce que** la branche libre (109) et la branche connectée (110) sont configurées pour recevoir un adaptateur (112) entre elles pour adapter une circonférence interne de la partie de montage (106).

10. Dispositif de déroulement (100) selon la revendication 9, **caractérisé en ce que** l'adaptateur (112) s'étend complètement sur la longueur de la partie de montage (106).

11. Dispositif de déroulement (100) selon la revendication 9 ou 10, **caractérisé en ce que** la section de fixation (106) présente un logement (113) dans lequel peut être logé un élément de fixation (114) formé sur l'adaptateur (112) pour relier de manière amovible l'adaptateur (112) à la section de fixation (106).

12. Dispositif de déroulement (100) selon l'une quelconque des revendications 1 à 11, **caractérisé en ce que** la plaque de base (101) présente un évidement (117) entre le tenon (102) et l'élément de déchirure (103).

13. Dispositif de déroulement (100) selon la revendication 12, **caractérisé en ce que** la plaque de base (101) présente un bord (118) à l'extrémité opposée à la branche (110), et **en ce que** l'évidement (117) s'étend jusqu'au bord (118).

14. Dispositif de déroulement (100) selon l'une quelconque des revendications 1 à 13, **caractérisé en ce que** le tenon (102) présente une collerette (119) qui est réalisée en un matériau élastique.

15. Dispositif de déroulement (100) selon l'une quelconque des revendications 1 à 14, **caractérisé en ce que** la plaque de base (101) et la section de fixation (106) comprenant la branche libre (109), la traverse (111) et la branche reliée (110) sont réalisées d'une seule pièce.
